# EUROPEAN PATENT SPECIFICATION

(11) **EP 1 476 013 B1**
(45) Date of publication and mention of the grant of the patent: **11.05.2011**
(21) Application number: 03704956.6
(22) Date of filing: 08.01.2003
(51) Int. Cl.: A01N 1/02, F25D 13/04, F25D 13/06

(54) **METHODS AND DEVICE FOR FREEZING AND THAWING BIOLOGICAL SAMPLES**
VERFAHREN UND GERÄT FÜR DAS EINFRIEREN UND AUFTAUEN VON BIOLOGISCHEN PROBEN
PROCEDES ET DISPOSITIF POUR CONGELER ET DECONGELER DES ECHANTILLONS BIOLOGIQUES

(30) Priority: 08.01.2002 US 345643 P; 27.06.2002 US 391575 P
(43) Date of publication of application: 17.11.2004
(60) Divisional application: 04023184.7
(73) Proprietor: Core Dynamics Limited, Hamilton HM EX (BM)
(72) Inventor: URI, Meir, Hashita 18910 (IL)
(74) Representative: Casey, Lindsay Joseph
(86) International application number: PCT/IL2003/000026
(87) International publication number: WO 2003/056919

(56) References cited:
- WO-A-01/78504
- WO-A-96/21351
- WO-A-98/10231

## Description

### FIELD OF THE INVENTION

This invention relates to methods and devices for the freezing and thawing of samples, including biological samples such as semen.

### BACKGROUND OF THE INVENTION

Cryopreservation of cells, tissues and organs has vast implications on numerous procedures. The cryopreserved samples can be used for grafting, *in vitro* manipulation (such as *in vitro* fertilization), research, etc. The rates of freezing and thawing a biological sample greatly affect the survival of the cells or tissue in the sample. When a biological sample containing living cells in a freezing solution is frozen, the first portion of the sample to freeze is the intercellular fluid. The formation of ice in the intercellular fluid increases the salt concentration there. If the sample is frozen too slowly, the high concentration of salt in the intercellular fluid may kill the cells, by osmotic shock or by chemical toxicity. Conversely, freezing the sample too rapidly may lead to the formation of intracellular ice crystals, which also kill the cell, by internal mechanical damage. In addition, the rate of cooling affects the morphology of the intercellular ice crystals. Morphologies such as closely packed needles also kill cells, by external mechanical damage. Thus, maximizing the survival rate of cells subjected to freezing and thawing requires careful control of the freezing process.

One method of freezing biological samples, which avoids ice crystal formation, is cooling the sample fast such that the intercellular and intracellular fluids vitrify instead of crystallizing as ice. However, if the rate of cooling is very fast, glass fractures may form within the sample at temperatures below its glass transition temperature due to thermal shock. Likewise, for thawing, vitrified samples are warmed as fast as they are cooled.

In a conventional slow-freezing method, a chamber is used in which the sample is introduced for freezing. Then, the temperature of the chamber is dropped in a controlled stepwise manner, thus exposing the sample to an external and gradual change in temperature. This method is based on using multidirectional (equiaxed) heat transfer to achieve a rate of temperature change in the sample that depends on the thermal conductivity and geometrical shape of the container and of the sample within it.

Additionally, when any liquid is cooled below its freezing point, it remains liquid, in an unstable super-cooled state, until freezing starts at randomly distributed nucleation sites and spreads throughout the entire volume of the liquid. When this process is uncontrolled the morphology of the ice can be irregular and be damaging to the sample.

A different technology for freezing is the "Multi-temperature gradient" (MTG) directional solidification, which is based on the invention disclosed in US 5,873,254. In this technology, the sample is moved at a constant velocity (V) through temperature gradients (G) so the cooling rate (G x V), ice front propagation are controlled and the velocity of the movement of the sample determines the morphology of the ice crystals formed within the sample. This method also enables the incorporation of controlled seeding into the freezing process.

The freezing of samples according to any of the known methods, even using accurate freezing rate control systems, is adapted for small samples that are 5 milliliters or less in volume. This is partially due to the fact that, in large samples, some parts of the sample (usually the outer zone or part thereof) may chill or warm faster than other parts. Thus, freezing and storage of semen is performed regularly using mini (¼ cc) or midi (½ cc) straws. Samples with volume of nearly 5 milliliter are usually frozen in plastic bags that are flattened during the preservation process, such as to have at least one dimension of the sample of less than 0.5 cm.

An exception to the above is a method described in co-pending PCT/IL02/00738, wherein the sample is agitated during freezing under the directional solidification process. Thus the rate of heat transfer within the sample is amplified, and the effect of the sample's bulk, morphology and heat transfer rate on the morphology of the forming ice crystals is reduced.

Cryopreservation of semen is a growing industry, utilized currently mostly in respect of bovine and human semen. In humans, sperm is cryopreserved mostly in sperm banks, for donation. In addition, human sperm is cryopreserved for use by the donor at a later time (e.g. in cases where infertility is expected or when the sperm count in any single ejaculate is too low to affect normal fertilization).

In the agricultural industry cryobanking of semen is used mostly for dairy cattle genetic breeding. First, semen is collected from young bulls in order to identify among them the preferred sires (proven bulls). The semen is used to inseminate heifers, which subsequently produce heifers. When these second-generation heifers are sufficiently grown they are inseminated and evaluated for milk production. During the time that this process requires (4-5 years) the bulls being evaluated may suffer injuries or death that might prevent their use as "proven bulls". Thus, semen is collected from the bulls and stored, only to be discarded for ca. 13 of every 14 bulls. In addition cryobanking of bull semen is an important backup for sufficient insemination doses in cases of disease, infertility, seasonal reduction is sperm production or mortality.

Various pre-freezing manipulations, such as sorting according to the sex chromosome of the sperm, can reduce the semen's freezability. In addition, in some species and in specific individuals, the sperm have a low freezability. For example, in cattle, where freezability is considered satisfactory, sperm may have 50-60% post thaw motility (PTM) and give rise to pregnancies after transcervical insemination. Contrarily, the semen of some species is chilling sensitive (e.g. ovine, porcine, canine, equine, and elephant semen) such that the PTM can be lower than 50% and post thaw pregnancy is reduced.

At times, there is need to remove the bulk of the seminal plasma prior to cryopreservation. This is done either in order to reduce the total volume of the sperm or in order to remove the plasma, which contains substances that reduce the sperm freezability. This may be achieved by centrifugation of the sample, washing of the sperm, and replacement of the removed plasma with a suitable extender. However, as the centrifugation in itself is known to damage sperm, the sperm of some species (e.g. equines or elephants) and some individuals cannot be effectively frozen in this manner. Other procedures for separation of the sperm from the plasma are expensive and/or time consuming, such as use of a separation column. These processes too are likely to reduce the freezability of the sperm.

Finally, it is noted that cryopreservation of other cells, and especially reproductive cells, may be useful for enhancement of reproduction and for the preservation of genetic material of zoo and wild animals that may face the danger of extinction.

### GLOSSARY

The following terms will have the meanings set aside them in the context of the present invention, unless the context clearly otherwise requires:
A *"sample"* means an amount of biological matter including cells and/or group of cells and/or bodily fluids and/or any constituents thereof. For example, a sample may comprise semen, oocytes (ova), blood, blood cells, blood constituents, germ cells, umbilical cord blood, plasma, zygotes and embryos.
A *"large sample"* means any sample that the minimal dimension of which, in each of two mutually perpendicular cross-sections, exceeds 0.5 centimeters. This minimal dimension may also exceed 1.6 centimetres and even 2.5 centimetres.
A *"large volume"* means any volume exceeding 5 millilitres. The volume may also exceed 12 millilitres and even 50 millilitres.
The term *"semen"* is taken to denote any sample containing sperm, with or without seminal plasma. It may be semen collected from any donor or from a donor treated in any manner that is intended to improve semen quality and/or freezability, such as a diet enriched with omega 9 fatty acids, Omega 3 fatty acids and/or with soy bean.
The term *"freezability"* means the ability of a biological sample to survive being frozen, namely to be frozen without suffering substantial damage. The damage to semen is expressed in such properties as sperm-count, motility, viability and fertility. The freezability of the semen can be evaluated accordingly by many ways including post thaw motility (PTM), staining for viability and the rates of successful fertilisation and impregnation.
The term *"extender"* means any solution or substance that, when added to a sample would improve its freezability.
The term *"whole ejaculate"* denotes most or all of the semen released from a single individual at a single ejaculation. The whole ejaculate may comprise essentially a whole ejaculate or a whole sperm rich fraction of an ejaculate. The *"sperm rich fraction"* of an ejaculate is the fraction of the ejaculate which contains most of the sperm of the ejaculate.
The term *"raw semen"* refers to the semen as it is collected, without any further manipulation such as centrifugation, or affinity separation. A sample of raw semen thus contains substantially the same proportion of sperm and seminal plasma that were in the raw semen.
An *"insemination quota"* is taken to mean a quantity of semen needed for a single procedure of insemination. In case of *in-vivo* insemination, such as for mammals (including humans) or avians, this means the amount of semen to be injected to a single female at a single time for the purpose of fertilisation. In case of *in-vitro* fertilization (IVF) this means the amount of semen to be used to fertilise one or more oocyts or ova. This is relevant for example to advanced fertilisation proceedings, such as human (or mammalian) IVF, where the female gametes are removed from the female and fertilised *in vitro.* This is also relevant for species where fertilisation takes place outside the body, such as fish and crustaceans.
Sperm is considered *"chilled",* when it is essentially immobile due to low temperature.

### DESCRIPTION OF THE INVENTION

The present invention provides a method and device for changing the temperature of a biological sample. The invention relates both to freezing and to thawing. The sample in the context of the invention may be a large sample and/or a sample having a large volume.

According to the invention, there is provided a method as specified in claim 1, and a device as specified in claim 14.

According to one aspect of the invention, referred herein as the isothermal-break method, there is provided a method for changing the temperature of a sample from an initial temperature via an intermediate temperature to a final temperature, one of the initial and final temperatures being above the freezing point of said sample and the other being below the freezing point, the minimal dimension of the sample in each of two mutually perpendicular cross-sections exceeding 0.5 centimeters, and at least one of the cross-sections having an outer zone and an inner zone, the method comprising:
(i) changing the temperature of the sample until the temperature of the sample in at least one part of the outer zone equals the intermediate temperature whilst the temperature of the sample in the inner zone or in another part of the outer zone, spaced from said one part, is different from said intermediate temperature;
(ii) further changing the temperature of said sample by subjecting it to the intermediate temperature until the temperature of the sample in said at least one cross-section is uniform and equals the intermediate temperature; and
(iii) changing the temperature of said sample until the majority of said sample is at the finial temperature.

The sample may be subjected in step (ii) to the intermediate temperature until not only its temperature in at least one cross-section is uniform and equals the intermediate temperature, but rather until the temperature of the whole sample, or most of the sample, equals said intermediate temperature, before being subjected to the change of temperature of step (iii).

It is by virtue of the present invention that cryopreservation of large samples and of samples having a large volume, may be successfully performed. This is preferably achieved by seeing that at least in most of the sample frozen or thawed according to the method of the invention has essentially the same temperature history, as all the other parts. This is of essence for example where the intermediate temperature is of a critical nature, namely that one rate of freezing (or thawing) is preferred between the initial and intermediate temperatures, and another is preferred between the intermediate and final temperatures.

The changing of the temperature of the sample in steps (i) and (iii) may be achieved by changing the ambient temperature to which said sample is exposed. This may be done for example by placement of the sample in a bath or chamber with an ambient temperature and removal of the sample when the internal temperature has sufficiently changed. The time for such removal may be established in many ways, including by direct observation of the sample, by direct measurement of the temperature within the sample, or by determination of the period of time necessary for the change to take place. The period of time depends on the properties of the sample, such as heat transfer and morphology and also on the ambient temperature to which the sample is exposed. Typically, the larger the difference between the intermediate temperature and the ambient temperature, a shorter period of time would be required. For example warming a frozen sample to an intermediate temperature of 38°C in a 50°C bath would take longer than in a 78°C bath. Nevertheless, care must be taken to avoid over-heating or over-cooling of a substantial portion of the sample (as the temperature of the outer zone of the sample would change quicker than the inner zone). This may be minimized for example by agitation of the sample during the changing of temperature.

The ambient temperature may also be changed, at least partially, in a gradual manner. This may be achieved at least partially by the gradual movement of said sample in the direction of a temperature gradient, similar to the MTG method. Thus step (i) or (iii) or any part thereof, may be achieved by the controlled movement of the sample along an ambient temperature gradient. In fact, the changing of the temperature in step (i) may be entirely achieved by moving the sample through a region with a temperature gradient from the initial temperature to the intermediate temperature. Step (iii) may be achieved for example, at least partially, by the transfer of the sample substantially at once into a region or chamber with the final temperature or by moving the sample through a region with a temperature gradient from the intermediate temperature to the final temperature.

The changing of temperature in step (ii) may be performed by placing the sample in a region with the intermediate temperature, said region having a pre-determined length along the direction of the movement of the sample. In one option, the length of this region is shorter than the sample. In such case, the region should at least be of such length that would allow the sample, while being moved along the region, to have the leading end reach the intermediate temperature by the time the same leading end leaves the region. The remainder of the sample is subjected to the intermediate temperature until the temperature of this remainder also equals the intermediate temperature. In this case, since the sample is exposed simultaneously to more than one region, the velocity of movement in all regions must be the same, to allow each and every part of the sample to undergo substantially the same thawing or freezing process.

Alternatively, the length of the region in step (ii) may be substantially equal to or greater than the length of the sample along the direction of its movement. In such embodiment, the sample may be moved in the region of step (i) and into the region of step (ii) with one velocity, and from the region of step (ii) and through the region of step (iii) in another velocity. The velocity of movement in step (ii) would in such case be variable, at least in the range between the velocities of movement in steps (i) and (iii).

Alternatively, step (ii) may comprise:
(a) moving the sample into the region with the intermediate temperature, until substantially the whole sample is within said region;
(b) pausing the movement of the sample within said region until the temperature of the sample is substantially uniform throughout the sample and equals the intermediate temperature; and
(c) moving the sample out of said region.

According to one embodiment of the invention, controlled seeding is allowed to take place. Thus, where the sample has a leading end along the direction of movement, step (i) may comprise:
(1) moving the leading end of the sample into a region with a temperature gradient from the initial temperature to the intermediate temperature;
(2) pausing the movement until seeding takes place at the leading end; and
(3) moving the sample through said region.

The seeding in step (2) may be achieved by introduction of liquid nitrogen to said leading end of the sample. Alternatively, this process can be achieved without manipulation of the sample, e.g. by pausing the sample for a sufficiently long time for seeding to begin at said leading end. Such time may be established in many ways, including by direct observation of the sample or by calculation of the period of time necessary for the change to take place according to the ambient conditions and the sample's properties.

Many different biological samples may be frozen or thawed according to the above isothermal-break method of the invention, including blood cells, plasma, blood products, semen, oocytes (ova) embryos, stem cells, zygotes and umbilical cord blood.

According to a different embodiment of the invention, a device is provided for changing the temperature of a sample, said device comprising:
- a track;
- a mechanism for moving the sample in a direction along said track.
- temperature control means for imposing a temperature gradient along a first area along said track;
- temperature control means for imposing a constant temperature along a second area along said track, such that the length of said second area along the track would be at least equal to the length of the sample along said track; and
- temperature control means for imposing a temperature gradient along a third area along said track;

The device according to this embodiment is not limited to the specific dimensions of the sample. Accordingly the device may be used for large samples, wherein the minimal dimension in each of two mutually perpendicular cross-sections, exceed 0.5 centimeters.

The sample may either be moved along the track at a constant velocity or at different velocities. This is applicable for example to the first and third areas of the track, wherein the velocity of movement within each area is preferably kept substantially constant. The velocity of movement within the second area may be variable, and the sample may be inserted in one velocity and removed in another velocity. In fact, the sample may even remain unmoved in the second area, before being removed.

According to a further embodiment of the invention, a device is provided for changing the temperature of a sample, the minimal dimension of the sample in each of two mutually perpendicular cross-sections exceeding 0.5 centimeters, said device comprising:
- a track;
- a mechanism for moving the sample in a direction along said track;
- temperature control means for imposing a temperature gradient along a first area along said track;
- temperature control means for imposing a constant temperature along a second area along said track, such that the length of said second area along the track would suffice to allow the sample, at each cross-section taken perpendicularly to said direction to reach the intermediate temperature by the time it is moved out of said second area; and
- temperature control means for imposing a temperature gradient along a third portion of said track.

According to yet another embodiment of the invention a device is provided for changing the temperature of a sample, the minimal dimension of the sample in each of each of two mutually perpendicular cross-sections exceeding 0.5 centimeters, said device comprising:
- a track;
- a mechanism for moving the sample along said track;
- temperature control means for imposing a temperature gradient along a first area along said track; and
- temperature control means for imposing a constant temperature along a second area along said track, such that the length of said second area along the track would be at least equal to the length of the sample along said track

The devices of the invention are not limited to ant specific mechanism for moving the sample along the track or according to the nature of the track. Neither is the device limited to the temperature control means, which for any given device may refrigerate or warm any area or part thereof.

It is specifically noted that the above devices of the invention may comprise additional parts. For example, an area along the track may be provided before the first area of the above devices, such that the temperature in said additional area is controlled at a constant temperature above the freezing point of the sample. A mechanism to introduce liquid nitrogen at the leading end (tip) of a sample may also be provided within the first area of any of the above devices, such that controlled seeding may be affected. Finally, one or more observation and recording devices, as well as various control devices, may also be incorporated in such devices.

According to yet another aspect of this invention a method is provided of freezing a whole ejaculate in a single test tube. The sperm may be frozen with or without the seminal plasma or part thereof, and an extender or any other substance may be added to the sample before or during freezing.

The method of freezing of the whole ejaculate may be performed according to the isothermal-break method of the invention. Nevertheless, the whole ejaculate may be frozen according to any other applicable method of freezing suitable for large samples, such as the method described in co-pending PCT/IL02/00738.

According to an aspect which is outside the scope of the invention, a method for double-freezing preservation of semen is provided comprising:
(A) freezing the semen in one or more aliquots;
(B) thawing at least one aliquots;
(C) dividing said thawed aliquot to smaller aliquots; and
(D) freezing at least one of said smaller aliquots.

An aliquot may be any amount of semen, including more than one ejaculate, a single ejaculate or less than a single ejaculate. It may be derived from one or more males. The aliquots of step (A) may be equal or different in size or contents one from the other. Likewise, the smaller aliquots of step (C) may be equal or different in size or contents one from the other. Additionally, a thawed aliquot may be diluted before being divided into smaller aliquots, such that the semen concentration would be lower in the smaller aliquots.

An example for use of this method is in the case of cryobanking of bull semen. The semen of young bulls may be collected and frozen, such that each ejaculate is kept in a single test-tube. When and if a bull is a "proven bull" the semen is thawed, diluted and refrozen (at least in part) in smaller aliquots. The size of the smaller aliquots would be such that each aliquot may be completely used after thawing. This would range from one to several insemination quotas. Such method enables the creation of a bank of "waiting bulls" in artificial insemination (AI) centers, which presently do not use semen cryobanking. In addition this method saves money in labor and consumables (filling, printing, liquid nitrogen for freezing and for storage etc.). The above aspects of the invention (freezing of whole ejaculates and double-freezing) may be particularly advantageous for the reduction of the cost of cryobanking of semen. Due to the limitation on the size of the frozen samples in the conventional methods, cryobanking of a large quantity of semen requires its division into a large number of straws prior to freezing. This is time- and labour-consuming, expensive and requires a lot of storage space and liquid nitrogen.

According to an additional embodiment of the double-freezing method of the invention, the sperm is divided in step (C) into aliquots in accordance with a trait of the semen, such as the sex chromosome of the sperm. The semen according to this embodiment is frozen first as unsorted semen and later thawed, sorted and frozen again for transfer and/or storage. This may allow sperm to be harvested, sorted, and used in different and even distant sites.

In another embodiment of the above double-freezing method, the semen is not completely thawed prior to its division. Instead, the thawing of step (B) is terminated when the sample reaches a desired temperature wherein the extra-cellular fluid of the sample is thawed while the sperm is still chilled. Thus, for example, the thawing may be terminated at 5°C. The sperm remains chilled and its metabolism remains very low so it is less susceptible to hazardous substances and depletes less energy resources than it would at room temperature. Additionally, when sperm is warmed from 0°C to room temperature, and when it is cooled from room temperature to 0°C, it undergoes membrane lipid phase transition. This transition may be hazardous to cells. Thus a benefit of termination of the thawing process while the sperm is still chilled may be that the sperm suffers less of the membrane lipid phase transition.

The termination of thawing may be achieved in any method, including the transfer of the sample to a solution at the desired temperature. The time for such transfer may be established in many ways, including by direct observation of the sample (e.g. to observe the process of ice melting), by direct measurement of the temperature within the sample, or by measurement or calculation of the period of time necessary for the change to take place.

The above double-freezing method is not limited to the isothermal-break method for freezing or thawing of the semen. The freezing and thawing steps may also be carried out according to any applicable method of freezing, such as those described in US 5,873,254 or co-pending PCT/IL02/00738.

According to a further aspect which is outside the scope of the present invention, a. method for preservation of semen is provided, said method comprising:
(I) adding of an extender to a raw semen sample; and
(II) freezing said sample.

This preservation method may be useful in order to avoid the need to remove seminal plasma from a sample before its freezing in cases where the seminal plasma comprises substances that impair the freezability of the semen. According to this method the seminal fluid is not removed and it is instead diluted with the added extended, such that its hindering effect is diminished.

This preservation method of the invention is not limited to the isothermal-break method for freezing of the semen. The preservation method may also be carried out according to any other applicable method of freezing, such as the methods described in US 5,873,254 or co-pending PCT7IL02/00738.

A need to remove the seminal plasma may also arise for example in cases where the semen comprises a large amount of seminal plasma. In such case, especially if liquid extender is to be added, the number of straws usually needed for freezing would be large, which entails a vast expenditure of time and money, and a plurality of straws may be needed to achieve an insemination quota. Accordingly, in such case freezing methods that allow freezing large samples (e.g. the isothermal-break method of the present invention and the methods of co-pending PCT/IL02/00738) may allow reduction of the costs involving handling of a large volume of semen.

According to yet another aspect which is outside the scope of the present invention, a method is provided of preservation of sperm comprising the freezing of sperm collected from more than one donor, as a mixture. The mixture so frozen may comprise more or less than one insemination quota.

This may be useful especially in cases where two or more females are serially inseminated. For example in sheep, the semen of a single ram (or a pool of semen from several rams) is used to inseminate numerous ewes. Likewise, in avians (such as turkey and fowl) the semen of several males is collected and used for the serial insemination of numerous females.

This aspect of freezing a mixture of semen derived from more than one male is not limited to the isothermal-break method for freezing of the semen. This may also be carried out according to any other applicable method of freezing, such as the methods described in US 5,873,254 or co-pending PCT/IL,02/00738. This may also be carried out according to any applicable method of freezing that allows the freezing of large samples.

It should be appreciated that the invention, in all of its aspects and embodiments, is not limited to the source of the biological sample, be it human or non-human. Non-limiting examples for non-human samples are mammalian (e.g. bovine, ovine, canine, feline, equine and porcine), avian (e.g. pigeon, dove, quail, turkey) and fish (e.g. salmonoid, carp, sea bream). In the case of mammalian sample, examples for animal sources are horses, ponies, donkeys, cattle, pigs, sheep and goats. It should further be noted that the samples are not limited to farming and agricultural animals, and is applicable also to zoo animals and wild animals, e.g. elephant, zebra, primates, etc. In this context, the invention is applicable to the preservation of samples from animals on the verge of extinction as well as for the assistance in reproduction.

It should further be appreciated that the invention, in all of its aspects and embodiments, is not limited to any certain volume of the sample (although in some aspects there is a limitation on the samples' minimal dimensions). The volume of sample of the invention may be a large volume as defined.

Additionally, the invention, in all of its aspects and embodiments may be performed also in large samples with the minimal dimension of which, in each of two mutually perpendicular cross-sections exceeds 1.6 centimeters and even 2.5 centimeters.

### DESCRIPTION OF THE DRAWINGS

In order to understand the invention and to see how it may be carried out in practice, a preferred embodiment will now be described, by way of non-limiting example only, with reference to the accompanying drawings, in which:
**Fig. 1** shows a schematic side view of a device of the present invention, based on thermally conductive blocks.
**Fig. 2** shows a schematic side view of another device of the present invention, based on thermally conductive blocks.
**Fig. 3** shows a schematic side view of yet another device of the present invention, based on thermally conductive blocks.
**Fig. 4** shows the percentage of impregnation of ewes using frozen semen and fresh semen.

### DESCRIPTION OF PREFERRED EMBODIMENTS OF THE INVENTION

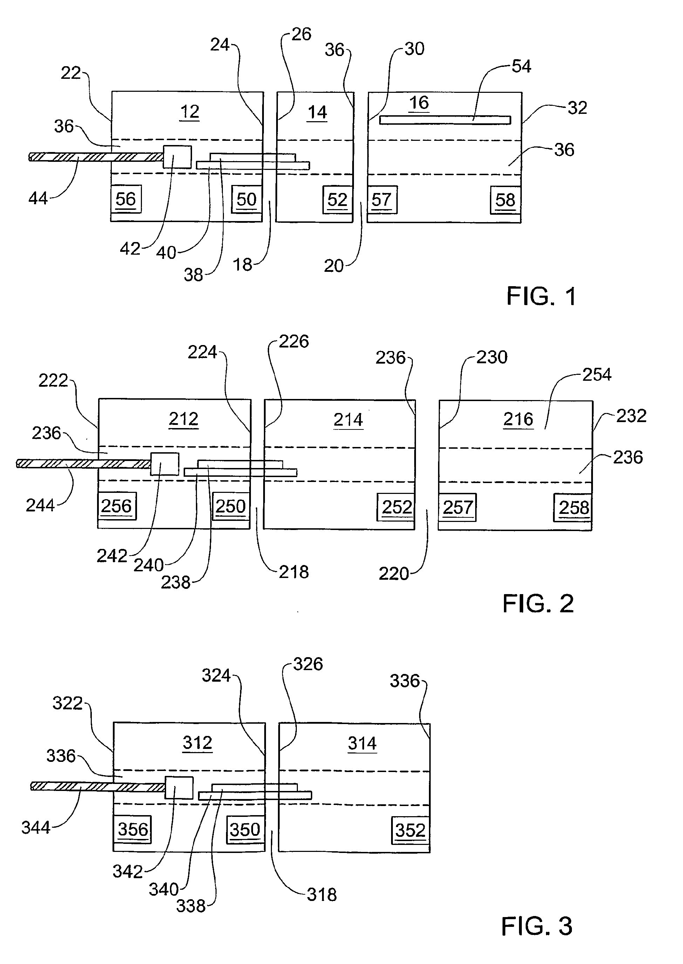

**Fig. 1** is a schematic side view of one embodiment of the device of the present invention. The device comprises three blocks 12, 14, and 16, of a thermally conductive material, such as brass, arranged in a line. Block 12 is about 22 centimeters long. Block 14 is about 5 centimeters long. Block 16 is about 22 centimeters long.

Blocks 12 and 14 are separated by a gap 18. Blocks 14 and 16 are separated by a gap 20. A tunnel 36, possibly of circular cross section, runs through blocks 12, 14, and 16. Tunnel 36 defmes a track along which a sled 40 is moved. Sled 40 preferably is made of a thermally conductive material, preferably brass, and bears one or more test tubes 38 that contain biological samples to be frozen or thawed. Test-tubes 38 may be tubes of circular cross section, about 10-12 centimeters long and with a diameter of 1.6 cm. Sled 40 is moved through tunnel 36 by a piston 42 to which is attached a helically threaded rod 44. Rod 44 is moved in the right direction of the drawing by a screw drive (not shown).

Blocks 12 and 14 include refrigerators 50 and 52. Blocks 12 and 16 include heaters 56, 57 and 58. Refrigerators 50 and 52 operate conventionally, by compressing and expanding cryogenic fluids. Heaters 56, 57 and 58 typically are electrical resistance heaters. Block 16 includes a channel 54 through which liquid nitrogen is circulated. Refrigerator 50 and heater 56 serve to impose a temperature gradient on the portion of tunnel 36 that runs from warm side 22 of block 12 to cold side 24 of block 12. Refrigerator 52 imposes a substantially constant temperature on block 14. The effect of circulation of the liquid nitrogen in channel 54 and heaters 57 and 58 is to impose a temperature gradient on the portion of tunnel 36 that runs from warm side 30 of block 16 to cold side 32 of block 16. The temperatures within blocks 12, 14, and 16 are monitored by thermocouples (not shown) and controlled by feedback loops (not shown) that include refrigerators 50 and 52 and heaters 56, 57 and 58.

Gaps 18 and 20 are typically 0-10 mm and may preferably be no wider than 2 centimeters. In that way, the tunnel extending through the blocks encloses substantially the entire track along which the biological samples move, essentially isolating the samples from the outside environment and facilitating the operation of the thermal gradients and plateaus of the blocks on the biological samples.

As a sample in test tubes 38 passes through block 12, it experiences an ambient gradient between the temperature of warm side 22 and the cold side 24 of said block (e.g. -5°C at the warm side 22 and -50°C at the cold side 24). When the leading end of the test tube 38 enters block 12 movement pauses for a short time to allow seeding to take place. Block 14 is kept at an intermediate temperature, e.g. -80°C. Block 16 can have a gradient be beginning at -85°C at the warm side 30 and getting colder towards the cold side 32.

When the sample containing test tube 38 is moved through the device it it enters block 12 after being slowly chilled to 5°C. Often this is performed with another block (not shown), kept at 5°C, through which the test tube 38 is moved.

**Fig. 2** is a schematic side view of another embodiment of the device of the present invention. Since the components of the device according to this embodiment are very similar to those of the device in Fig. 1, the same reference numerals are used herein, pre-fixed by the digit 2 to denote the same components. The following will detail only the differences between the devices:

According to the present embodiment, the length of block 214 is 12 centimeters long, which is typically the same as or longer than the length of the test tube 238. Accordingly, the following protocol may be used with the device of this embodiment:

The temperatures of the blocks are the same as mentioned above for Fig. 1. The sample in test tubes 328 is moved into block 212 and once the leading end is inserted, it pauses for 30-60 seconds to allow seeding to take place at said leading end. It is then moved through the block at 1 mm/sec, until the whole sample is within block 214. Movement is typically ceased for 20-30 seconds and then resumed at the above or a different velocity until test tube 23 8 fully exits block 216.

**Fig. 3** is a schematic side view of yet another embodiment of the device of the present invention. Since the components of the device according to this embodiment are very similar to those of the device in Fig. 2, the same reference numerals are used herein, pre-fixed by the digit 3 to denote the same components. The following will detail only the differences between the device of this embodiment and the device of Fig. 2:

According to the present embodiment, block 16 of Fig. 2 was removed from the device. The test tubes 338, typically being of a length of 10-12 cm and a diameter of 1.6 cm are moved through the device of the present embodiments exactly as described above for Fig. 2. After the whole sample is within block 314, and movement has been ceased for 20-30 seconds the test tube 338 is removed, for example, for storage in liquid nitrogen.

In an alternative device (not shown), blocks 312 and 314 are replaced by a single block. Such blocks are equipped with temperature control means that provide a gradient between the warm end of the block to some place along the track (approximately at the length of block 312). From there the temperature is maintained constant. Accordingly, the test tube 338 would experience first a gradient (e.g. -5° to -80°C) followed by a constant temperature at -80°C. The length along the track kept at the constant temperature would be equal to the length of block 314.

Following are several examples of embodiments of the present invention.

### Example 1- Cryopreservation of Bovine Semen

### Post Thaw Motility (PTM) Experiments:

Semen from two bulls was collected and tested for semen concentration and motility (>70%) before dilution. AndroMed® (minitub, Hauptstrabe, Germany) was used to dilute the semen to a concentration of 15x10⁶ sperm/ml.

In the test sample, whole ejaculates were each put into a 12 ml test tube (with a diameter of 16mm). The tubes were frozen in a gradient from 5°C to -50°C at 55°C/min (1 mm/sec) and maintained at -80°C for app. 60 seconds using the MTG 516 device (manufactured by IMT Interface Multigrad Technology, Israel). The tubes were then thawed to room temperature and divided to aliquots in mini-straws (0.25 cc) each equaling one insemination quota. The aliquots were refrozen in a conventional method.

In the control samples the diluted semen was divided to aliquots in mini-straws (0.25 cc) each equaling an insemination quota and frozen only once, in a conventional method.

The thawed sperm (test and control) was tested for PTM and gave the following results:
**Test sample**: after the first freezing (in the 12 ml tubes) PTM was 75±5% (which was 90-100% of the pre-freeze motility of the semen from the same ejaculate). After thawing to room temperature and re-freezing in mini-straws PTM was 50±5%.
**Control sample**: showed 60±10% post thaw motility after being frozen once, in mini-straws.

In a second experiment, whole ejaculates were collected from 4 bulls and frozen, each in 9 ml test tubes using an MTG 516 device. Thawing was performed in 60°C water bath for 15 sec until ice melting was observed transferred into a cold (5°C) solution and immediately refrozen in regular ¼ cc straws in a conventional protocol, over liquid nitrogen (LN) vapor. The results are shown in Table I.

**Table I - Post Thaw motility of Bovine Sperm**

| **Bull name** | **Pre-freezing g Motility** | **PTM after first freezing (9ml tubes)** | **PTM after second (0.25 ml straws)** |
|---|---|---|---|
| **Sufon** | 90% | 80% | 60% |
| **Lukon** | 95% | 80% | 60% |
| **DanDan** | 90% | 70% | 50% |
| **Avsha** | 85% | 70% | 50% |
| **Average** | **90%** | **75%** | **55%** |

### Pregnancy Experiment:

Some of the sperm from the above first experiment (where the semen was thawed to room temperature during the double-freezing process) was used also for field trials in which cows were inseminated. 105 cows were inseminated using the test sample (after double-freezing), and another 123 cows were inseminated with control semen (frozen only once).

The test semen (after double-freezing) and the control semen gave similar pregnancy rates - 44% (47/105) for the double-freezing group in comparison to 45.5% (56/123) for the control group. This shows that semen can undergo double-freezing in accordance with the present invention without effecting sperm fertility.

By cryobanking whole ejaculates of bull semen it is possible to save freezing and storage expenses. The procedure is good also for semen of males of other species such as stallion and boar.

Artificial insemination (AI) centers that usually have a bank of 10,000 frozen straws per bull, which are made from the semen obtained in 25 ejaculates (400 straws/ejaculate). These 10,000 straws would fit into 13 goblets (750 straw/goblet). In comparison, according to the present invention, when a whole ejaculate is frozen in a 12 ml test tube, the 25 ejaculates will be fit in 25 test tubes which will be stored only in 2 goblets. This means that 6.5 times more goblets are required using straws in comparison to the freezing of whole ejaculates in single test tubes.

Accordingly, the present invention enables the creation of a bank of "waiting bulls" in some of the AI centers, which presently do not use semen cryobanking. In addition this method will save money in labor and consumables (filling, printing, LN for freezing and for storage etc.).

### Example 2 - Cryopreservation of Equine Semen

Semen was collected from 3 stallions. Each ejaculate was split and one part was centrifuged and diluted with 20% egg yolk glucose freezing extender containing 5% glycerol to 150X10⁶ml⁻¹. Another part was left non-centrifuged, and was diluted with the same extender at the proportion of 1:11 (semen/extender). Semen diluted to 150X10⁶ml⁻¹ was frozen in mini-straws as two controls, one using a Planer device (Planer, UK) wherein the semen was frozen over liquid nitrogen at 50-60°C/min, and the other using MTG525 machine (manufactured by IMT Interface Multigrad Technology Ltd. Israel, according to the manufacturer's manual).

The remaining semen was frozen in 12ml tubes with a gradient between 5 °C and -50° C, after which the tubes were left in -80°C for 60 seconds using the MTG 516 device.

Straws were evaluated for progressive motility after thawing at 37°C for 30 seconds. Tubes were evaluated after holding in air for 140 seconds, then plunging into water at 37°C until completely thawed (approximately 2 minutes).

**Table II - Post Thaw motility of Equine Sperm**

| **Stallion** | **Mini-straw in Planer Device** | **Mini-straw in MTG525** | **12 ml Tube 50X10⁶ml⁻¹ after centrifugation** | **12 ml Tube 1:11 no centrifugation** |
|---|---|---|---|---|
| **Vedonau** | 50% | 55% | 55% | 70% |
| **Fantast** | 60% | 60% | 65% | 70% |
| **Nelson** | 40% | 45% | 55% | 60% |
| **Average** | **50%** | **53%** | **58%** | **67%** |

Dilution of the sperm at 1:11 ratio gave better PTM results over centrifugation. At this dilution rate, to deliver the correct dose of semen requires a very large insemination volume (circa 50ml). It has been shown that large insemination volume enhances uterine contractions in mares, and thus facilitates fertilization. Therefore, the large volume of freezing often required in the case of dilution without centrifugation may prove advantageous in both in terms of centrifugation elimination and enhanced uterine contraction in the mare.

### Example 3 - Cryopreservation of Equine Semen

Semen was collected from stallions. Each ejaculate was centrifuged, washed and diluted with 20% egg yolk glucose freezing extender containing 5% glycerol.

Control samples were loaded in mini-straws and were frozen in a Planer device (50-60°C/sec). Tubes were frozen using the MTG 516 device with a gradient between 5° and -50°C with a 20 second pause when the leading end (tip) was at -5°C for seeding to take place therein, after which the tubes were left in -80°C for ca. 60 seconds.

Straws were thawed at 37°C for 30 seconds, and tubes were thawed by holding in air for 140 seconds, then plunging into water at 37°C until completely thawed (approximately 2 minutes). The thawed samples were evaluated for progressive motility (PTM), stained for viability (AO/PI) and subjected to an osmotic response test (ORT) and assayed for motility after 10 min incubation at 37°C. The Fail/Pass results in the following are related to the post-incubation motility, namely 20% or more are considered a passing result and less than 20% is a failing result.

**Table III - Freezability of Equine Sperm**

| Stallion | Pre-Freeze motility | Planer Straw | | | | MTG Tube | | | |
|---|---|---|---|---|---|---|---|---|---|
| | | % PTM | AO/PI %live | ORT | Fail/ Pass | % PTM | AO/PI %live | ORT | Fail/ Pass |
| Libra-K | 80% | 20% | 40% | 33% | Fail | 35% | 44% | 29% | Pass |
| Samhire | 60% | 3% | 32% | 23% | Fail | 60% | 49% | 46% | Pass |
| Libra-K | 80% | 20% | 43% | 34% | Fail | 40% | 57% | 46% | Pass |
| Mill Law | 40% | 10% | 35% | 26% | Fail | 30% | 42% | 36% | Pass |
| Jester | 90% | 30% | 29% | 20% | Fail | 40% | 40% | 23% | Pass |
| Rob Roy | 90% | 80% | 75% | 56% | Fail | 80% | 78% | 64% | Pass |
| Pall Mall | 70% | 25% | 32% | 16% | Fail | 35% | 38% | 26% | Pass |
| Jester | 60% | 30% | 34% | 23% | Fail | 50% | 53% | 43% | Pass |
| Dramiro | 70% | 20% | 24% | 11% | Fail | 40% | 49% | 42% | Pass |
| Rubek | 60% | 35% | 39% | 21% | Pass | 50% | 44% | 44% | Pass |
| Rubek | 60% | 50% | 50% | 36% | Pass | 60% | 49% | 40% | Pass |
| Secundus | 70% | 35% | 24% | 22% | Pass | 50% | 54% | 43% | Pass |
| Schiller | 90% | 60% | 44% | 35% | Pass | 60% | 65% | 51% | Pass |
| Ludwig | 80% | 35% | 35% | 29% | Pass | 40% | 37% | 29% | Pass |
| Schiller | 80% | 40% | 38% | 28% | Pass | 45% | 46% | 28% | Pass |
| Secundus | 80% | 40% | 43% | 36% | Pass | 50% | 51% | 38% | Pass |
| mean | 72.5% | 33.3% | 38.5% | 28.1% | - | 47.8% | 49.8% | 39.3% | - |
| mean F-P* | 71.1% | 26.4% | 38.2% | 27.0% | - | 45.6% | 50.1% | 39.5% | - |
| mean P-P** | 74.3% | 42.1% | 38.8% | 29.5% | 7/16 pass | 50.7% | 49.5% | 39.0% | 16/16 Pass |

| | | | | | | | | | |
|---|---|---|---|---|---|---|---|---|---|
| * The average results relate to the first 9 samples wherein the sperm failed in the Planer straw results and passed in the MTG Tube results ** The average results relate to the last 7 samples wherein the sperm passed in both results. | | | | | | | | | |

### Example 4 - Cryopreservation of Fowl Semen

Sperm was collected from six fowl twice a week for two-month (6 times altogether). The semen was evaluated individually for viability and motility using SQA (Sperm Quality Analyzer) and microscopic evaluation. Only good sperm were used (i.e. >50% motility and SMI (Sperm Motility Index)). The semen was pooled and exposed to Minitub dilution medium containing 10% glycerol (1:1). Freezing was done using ALON 1000 (manufactured by IMT Interface Multigrad Technology Ltd. Israel) in 5ml straws. After pre-incubation for 2 hours at 4°C the straws were loaded at the 5°C block and frozen with interface velocity of 1.5 mm/sec from 5°C to -50°C. When the leading end (tip) of the straw reached the -5°C point, movement was paused for 60 seconds and then resumed at the same speed. The tubes were then maintained in ca. -80°C for ca. 20 seconds and were then transferred to liquid nitrogen (LN). Thawing: the straws were warmed at 55°C water bath for 22 seconds following with 30 seconds at 38°C. Evaluated using SQA, microscopic evaluation and fluorescent staining for membrane integrity.

**Table IV - Freezability of Fowl Sperm**

| | Pool 1 | Pool 2 | Pool 3 | Pool 4 | Pool 5 | Pool 6 | Average |
|---|---|---|---|---|---|---|---|
| Pre-freezing | 58% | 76% | 56% | 80% | 70% | 80% | 70% |
| Post thaw | 43% | 34% | 30% | 40% | 55% | 50% | 42% |
| Normalized motility | 74% | 44% | 53% | 50% | 78% | 63% | 60% |

### Example 5 - Cryopreservation of Ovine Semen

Semen was collected from 5 rams, and evaluated for motility by microscopy and SQA. All samples displayed 80-90% motility, and were subsequently diluted with New Zealand™ extender to ca. 450 million cells/ml. The semen was cooled slowly to 5°C (1°C/min), and divided into 5 ml tubes. The tubes were frozen in ALON 1000 (manufactured by IMT Interface Multigrad Technology Ltd. Israel) to -50°C at 0.3 mm/sec. The samples were exposed to -50°C at least 20-60secs. Then the samples were transferred to liquid nitrogen for storage. Thawing of the tubes for use was by incubating the straws in a 75°C bath for 22 seconds and then 30 seconds in a 38°C bath.

Five months after freezing, two tubes from each ram were thawed and evaluated for motility. Six months after freezing the samples were thawed and used for cervical insemination of 40 ewes (each tube was used to inseminate 10 females).

In a control experiment, fresh semen was collected from the same rams and used, in two different dilutions (1:1 and 1:10), to inseminate 50 ewes. Approximately 2 months after inseminations, the ewes were tested by ultrasound for pregnancy.

**PTM Results**: The thawed semen displayed better PTM for rams 2808 and 6750 (60-70% PTM) than for rams 5539 and 5928 (ca. 50%). The PTM of ram 6570 was only 30-45%.

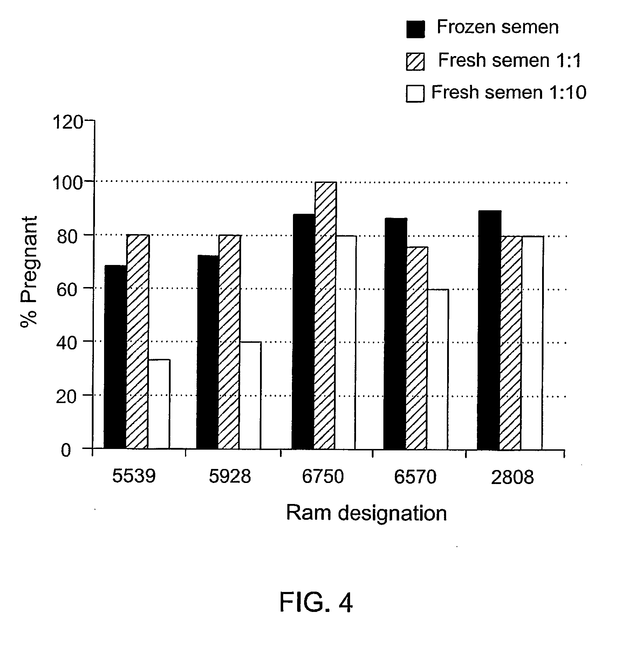

**The Insemination Results** are shown in Fig. 4. The pregnancy rate of the thawed semen and the fresh semen was comparable, and in fact the frozen semen provided better results than the fresh semen that was diluted 1:10.

## Claims

1. A method for changing the temperature of a sample from an initial temperature via an intermediate temperature to a final temperature, one of the initial and final temperatures being above the freezing point of said sample and the other being below the freezing point, the minimal dimension of the sample in each of two mutually perpendicular cross-scctions exceeding 0.5 centimeters, and at least one of the cross-sections having an outer zone and an inner zone, the method comprising:
(i) changing the temperature of the sample by subjecting it to a temperature gradient from the initial temperature to the intermediate temperature until the temperature of the sample in at least one part of the outer zone equals the intermediate temperature whilst the temperature of the sample in the inner zone or in another part of the outer zone, spaced from said one part, is different from said intermediate temperature;
(ii) further changing the temperature of said sample by subjecting it to the intermediate temperature until the temperature of said sample in at least one cross-section is uniform and equals the intermediate temperature; and
(iii) changing the temperature of said sample until the majority of said sample is at the final temperature.

2. A method as claimed in Claim 1, wherein said sample is subjected in step (ii) to said intermediate temperature until the temperature of the sample equals said intermediate temperature.

3. A method as claimed in any one of Claims 1 or 2, wherein the changing of the temperature in step (i) is achieved by moving the sample through a region with the temperature gradient from the initial temperature to the intermediate temperatures, and the changing of the temperature in step (iii) is achieved by moving the simple through a region with a temperature gradient from the intermediate temperature to the final temperature.

4. A method as claimed in Claim 3, wherein said changing of the ambient temperature is at least partially gradual and is achieved at least partially by the gradual movement of said sample in the direction of a temperature gradient.

5. A method as claimed in Claim 4, wherein the changing of temperature in step (ii) is performed by placing said sample in a region with the intermediate temperature, said region having a length along the direction of the movement of said sample and said length is not less the length of the sample along said direction of movement.

6. A method as claimed in Claim 5, wherein the changing of the temperature in step (i) is achieved by moving the sample through a region with a temperature gradient from the initial temperature to the intermediate temperature, and the changing of the temperature in step (iii) is achieved by moving the sample through a region with a temperature gradient from the intermediate temperature to the final temperature.

7. A method as claimed in any one of Claims 5 or 6, wherein the sample has a leading end along the direction of movement and step (i) comprises:
(1) moving the leading end of the sample into a region with a temperature gradient from the initial temperature to the intermediate temperature;
(2) pausing the movement until seeding takes place at the leading end; and
(3) moving the sample through said region.

8. A method as claimed in Claim 7, wherein the seeding in step (2) is achieved by introduction of liquid nitrogen to said leading end of the sample.

9. A method as claimed in any one of Claims 5 to 8, wherein step (ii) comprises:
(a) Moving the sample into the region with the intermediate temperature, until substantially the whole sample is within said region;
(b) Pausing the movement of the sample within said region until the temperature of the sample is substantially uniform throughout the sample and equals the intermediate temperature; and
(c) Moving the sample out of said region.

10. A method as claimed in any of Claims 5 to 9, wherein the volume of the sample exceeds 5 milliliters.

11. A method as claimed in any of Claims 5 to 10, wherein the volume of the sample is 12 milliliters or more.

12. A method as claimed in any of Claims 5 to 11, wherein the volume of the sample is 50 milliliters or more.

13. A method according to any one of Claims 1 to 9 wherein the sample comprises blood cells, or plasma or one or more embryos, or semen.

14. A device for changing the temperature of a sample from an initial temperature via an intermediate temperature to a final temperature, one of the initial and final temperatures being above the freezing point of said sample and the other being below the freezing point, the minimal dimension of the sample in each of two mutually perpendicular cross-sections exceeding 0.5 centimeters, said device comprising:
- a track;
- a mechanism for moving the sample in a direction along said track;
- temperature control means for imposing a temperature gradient along a first area along said track;
- temperature control means for imposing a constant temperature along a second area along said track, such that the length of said second area along the track would suffice to allow the sample, at each cross-section taken perpendicularly to said direction to reach the intermediate temperature by the time it is moved out of said second area; and
- temperature control means for imposing a temperature gradient along a third area of said track.

15. A device as claimed in Claim 14, wherein the sample has a volume of at least 5 milliliters.

16. A device as claimed in claim 14 or 15, wherein the sample has a volume exceeding 12 milliliters.

17. A, device as claimed in any one of claims 14-16, wherein the sample has a volume exceeding 50 milliliters.

18. A device according to Claim 14, wherein the mechanism for moving the sample along said track is capable of moving the sample at a constant velocity, along the track in said first and third areas.

19. A device according to Claim 14, wherein the mechanism for moving the sample along said track is capable of moving the sample along the track in said first and third areas at different velocities one from the other.

20. A device according to Claim 14, wherein the mechanism for moving the sample along said track is capable of stopping the movement when the sample is within the second area and later resuming the movement.

21. A device according to any one of Claims 14 to 20, wherein the initial temperature is above the freezing point of the sample and the final temperature is below said freezing point.

## Patentansprüche

1. Verfahren zur Veränderung der Temperatur einer Probe von einer Anfangstemperatur über eine Zwischentemperatur auf eine Endtemperatur, wobei eine der Anfangs- und Endtemperatur über dem Gefrierpunkt der genannten Probe liegt und die andere unter dem Gefrierpunkt liegt, die minimale Abmessung der Probe in jedem von zwei zueinander senkrechten Querschnitten mehr als 0,5 Zentimeter beträgt und mindestens einer der Querschnitte einen äußeren Bereich und einen inneren Bereich hat, wobei das Verfahren Folgendes umfasst:
(i) Verändern der Temperatur der Probe durch das Aussetzen dieser gegenüber einem Temperaturgradienten von der Anfangstemperatur bis zur Zwischentemperatur bis die Temperatur der Probe in mindestens einem Teil des äußeren Bereichs gleich der Zwischentemperatur ist, während sich die Temperatur der Probe im inneren Bereich oder in einem anderen Teil des äußeren Bereichs, in einem Abstand zum einen genannten Teil, von der genannten Zwischentemperatur unterscheidet;
(ii) weiteres Verändern der Temperatur der genannten Probe durch das Aussetzen dieser gegenüber der Zwischentemperatur bis die Temperatur der genannten Probe in mindestens einem Querschnitt einheitlich ist und gleich der Zwischentemperatur ist; und
(iii) Verändern der Temperatur der genannten Probe bis der Großteil der genannten Probe die Endtemperatur besitzt.

2. Verfahren nach Anspruch 1, worin genannte Probe im Schritt (ii) genannter Zwischentemperatur ausgesetzt wird bis die Temperatur der Probe gleich der genannten Zwischentemperatur ist.

3. Verfahren nach einem der Ansprüche 1 oder 2, worin das Verändern der Temperatur im Schritt (i) durch Bewegen der Probe durch einen Bereich mit dem Temperaturgradienten von der Anfangstemperatur bis zur Zwischentemperatur erreicht wird und das Verändern der Temperatur im Schritt (iii) durch Bewegen der Probe durch einen Bereich mit einem Temperaturgradienten von der Zwischentemperatur bis zur Endtemperatur erreicht wird.

4. Verfahren nach Anspruch 3, worin das genannte Verändern der Umgebungstemperatur zumindest teilweise graduell erfolgt und zumindest teilweise durch die graduelle Bewegung von genannter Probe in Richtung eines Temperaturgradienten erreicht wird.

5. Verfahren nach Anspruch 4, worin das Verändern der Temperatur im Schritt (ii) durch Platzieren der genannten Probe in einen Bereich mit der Zwischentemperatur erfolgt, wobei genannter Bereich eine Länge entlang der Richtung der Bewegung der genannten Probe hat und genannte Länge nicht weniger als die Länge der Probe entlang der genannten Richtung der Bewegung beträgt.

6. Verfahren nach Anspruch 5, worin das Verändern der Temperatur im Schritt (i) durch Bewegen der Probe durch einen Bereich mit einem Temperaturgradienten von der Anfangstemperatur bis zur Zwischentemperatur erreicht wird und das Verändern der Temperatur im Schritt (iii) durch Bewegen der Probe durch einen Bereich mit einem Temperaturgradienten von der Zwischentemperatur bis zur Endtemperatur erreicht wird.

7. Verfahren nach einem der Ansprüche 5 oder 6, worin die Probe ein führendes Ende entlang der Richtung der Bewegung hat und Schritt (i) Folgendes umfasst:
(1) Bewegen des führenden Endes der Probe in einen Bereich mit einem Temperaturgradienten von der Anfangstemperatur bis zur Zwischentemperatur;
(2) Anhalten der Bewegung bis das Seeding am führenden Ende auftritt; und
(3) Bewegen der Probe durch genannten Bereich.

8. Verfahren nach Anspruch 7, worin das Seeding im Schritt (2) durch Einführung von flüssigem Stickstoff in genanntes führendes Ende der Probe erreicht wird.

9. Verfahren nach einem der Ansprüche 5 bis 8, worin Schritt (ii) Folgendes umfasst:
(a) Bewegen der Probe in den Bereich mit der Zwischentemperatur bis sich im Wesentlichen die gesamte Probe im genannten Bereich befindet;
(b) Anhalten der Bewegung der Probe im genannten Bereich bis die Temperatur der Probe im Wesentlichen in der gesamten Probe einheitlich ist und gleich der Zwischentemperatur ist; und
(c) Bewegen der Probe aus dem genannten Bereich.

10. Verfahren nach einem der Ansprüche 5 bis 9, worin das Volumen der Probe mehr als 5 Milliliter beträgt.

11. Verfahren nach einem der Ansprüche 5 bis 10, worin das Volumen der Probe 12 Milliliter oder mehr beträgt.

12. Verfahren nach einem der Ansprüche 5 bis 11, worin das Volumen der Probe 50 Milliliter oder mehr beträgt.

13. Verfahren nach einem der Ansprüche 1 bis 9, worin die Probe Blutzellen oder Plasma oder einen oder mehrere Embryonen oder Sperma umfasst.

14. Vorrichtung zur Veränderung der Temperatur einer Probe von einer Anfangstemperatur über eine Zwischentemperatur auf eine Endtemperatur, wobei eine der Anfangs- und Endtemperatur über dem Gefrierpunkt der genannten Probe liegt und die andere unter dem Gefrierpunkt liegt, die minimale Abmessung der Probe in jedem von zwei zueinander senkrechten Querschnitten mehr als 0,5 Zentimeter beträgt, wobei die genannte Vorrichtung Folgendes umfasst:
- eine Führungsbahn;
- eine Mechanik zur Bewegung der Probe in Richtung entlang genannter Führungsbahn;
- Temperaturkontrollmittel zum Auferlegen eines Temperaturgradienten entlang einem ersten Bereich entlang genannter Führungsbahn;
- Temperaturkontrollmittel zum Auferlegen einer konstanten Temperatur entlang einem zweiten Bereich entlang genannter Führungsbahn, sodass die Länge von genanntem zweiten Bereich entlang der Führungsbahn ausreichen würde, um zu ermöglichen, dass die Probe an jedem Querschnitt, der senkrecht zur genannten Richtung genommen wird, die Zwischentemperatur bis zu dem Zeitpunkt erreicht, wenn sie aus genanntem zweiten Bereich bewegt wird; und
- Temperaturkontrollmittel zum Auferlegen eines Temperaturgradienten entlang einem dritten Bereich von genannter Führungsbahn.

15. Vorrichtung nach Anspruch 14, worin die Probe ein Volumen von mindestens 5 Milliliter hat.

16. Vorrichtung nach Anspruch 14 oder 15, worin die Probe ein Volumen von mehr als 12 Milliliter hat.

17. Vorrichtung nach einem der Ansprüche 14-16, worin die Probe ein Volumen von mehr als 50 Milliliter hat.

18. Vorrichtung nach Anspruch 14, worin die Mechanik zur Bewegung der Probe entlang der genannten Führungsbahn die Probe bei einer konstanten Geschwindigkeit entlang der Führungsbahn im genannten ersten und dritten Bereich bewegen kann.

19. Vorrichtung nach Anspruch 14, worin die Mechanik zur Bewegung der Probe entlang der genannten Führungsbahn die Probe entlang der Führungsbahn im genannten ersten und dritten Bereich bei voneinander unterschiedlichen Geschwindigkeiten bewegen kann.

20. Vorrichtung nach Anspruch 14, worin die Mechanik zur Bewegung der Probe entlang der genannten Führungsbahn die Bewegung, wenn die Probe im zweiten Bereich ist, anhalten und später die Bewegung wieder aufnehmen kann.

21. Vorrichtung nach einem der Ansprüche 14 bis 20, worin die Anfangstemperatur über dem Gefrierpunkt der Probe liegt und die Endtemperatur unter dem genannten Gefrierpunkt liegt.

## Revendications

1. Méthode destinée à modifier la température d'un échantillon depuis une température initiale en passant par une température intermédiaire jusqu'à une température finale, l'une parmi les températures initiale et finale étant au-dessus du point de congélation dudit échantillon et l'autre étant en dessous du point de congélation, la dimension minimale de l'échantillon dans chacune de deux sections transversales mutuellement perpendiculaires dépassant 0,5 centimètre, et au moins l'une des sections transversales ayant une zone extérieure et une zone intérieure, la méthode comprenant les étapes consistant à :
(i) modifier la température de l'échantillon en le soumettant à un gradient de température depuis la température initiale jusqu'à la température intermédiaire jusqu'à ce que la température de l'échantillon dans au moins une partie de la zone extérieure soit égale à la température intermédiaire alors que la température de l'échantillon dans la zone intérieure ou dans une autre partie de la zone extérieure se trouvant à distance de ladite une partie est différente de ladite température intermédiaire ;
(ii) modifier en outre la température dudit échantillon en le soumettant à la température intermédiaire jusqu'à ce que la température dudit échantillon dans au moins une section transversale soit uniforme et égale à la température intermédiaire ; et
(iii) modifier la température dudit échantillon jusqu'à ce que la majeure partie dudit échantillon soit à la température finale.

2. Méthode selon la revendication 1, dans laquelle ledit échantillon est soumis à l'étape (ii) à ladite température intermédiaire jusqu'à ce que la température de l'échantillon soit égale à ladite température intermédiaire.

3. Méthode selon l'une quelconque des revendications 1 ou 2, dans laquelle la modification de la température à l'étape (i) est réalisée en déplaçant l'échantillon sur une région présentant le gradient de température depuis la température initiale jusqu'à la température intermédiaire, et la modification de la température à l'étape (iii) est réalisée en déplaçant l'échantillon sur une région présentant un gradient de température depuis la température intermédiaire jusqu'à la température finale.

4. Méthode selon la revendication 3, dans laquelle ladite modification de la température ambiante est au moins en partie graduelle et est réalisée au moins en partie par le mouvement graduel dudit échantillon en direction d'un gradient de température.

5. Méthode selon la revendication 4, dans laquelle la modification de la température à l'étape (ii) est réalisée en plaçant ledit échantillon dans une région ayant la température intermédiaire, ladite région ayant une longueur sur la direction du mouvement dudit échantillon et ladite longueur n'est pas inférieure à la longueur de l'échantillon sur ladite direction de mouvement.

6. Méthode selon la revendication 5, dans laquelle la modification de la température à l'étape (i) est réalisée en déplaçant l'échantillon sur une région présentant un gradient de température depuis la température initiale jusqu'à la température intermédiaire, et la modification de la température à l'étape (iii) est réalisée en déplaçant l'échantillon sur une région présentant un gradient de température depuis la température intermédiaire jusqu'à la température finale.

7. Méthode selon l'une quelconque des revendications 5 ou 6, dans laquelle l'échantillon présente une extrémité de tête le long de la direction de mouvement et l'étape (i) comprend les actions consistant à :
(1) déplacer l'extrémité de tête de l'échantillon à l'intérieur d'une région présentant un gradient de température depuis la température initiale jusqu'à la température intermédiaire ;
(2) interrompre le mouvement jusqu'à ce que l'ensemencement se produise au niveau de l'extrémité de tête ; et
(3) déplacer l'échantillon sur ladite région.

8. Méthode selon la revendication 7, dans laquelle l'ensemencement de l'étape (2) est réalisé par l'introduction d'azote liquide à ladite extrémité de tête de l'échantillon.

9. Méthode selon l'une quelconque des revendications 5 à 8, dans laquelle l'étape (ii) comprend les étapes consistant à :
(a) déplacer l'échantillon dans la région présentant la température intermédiaire jusqu'à ce que l'ensemble de l'échantillon soit essentiellement à l'intérieur de ladite région ;
(b) interrompre le mouvement de l'échantillon à l'intérieur de ladite région jusqu'à ce que la température de l'échantillon soit substantiellement uniforme sur tout l'échantillon et soit égale à la température intermédiaire ; et
(c) déplacer l'échantillon hors de ladite région.

10. Méthode selon l'une quelconque des revendications 5 à 9, dans laquelle le volume de l'échantillon dépasse 5 millilitres.

11. Méthode selon l'une quelconque des revendications 5 à 10, dans laquelle le volume de l'échantillon est de 12 millilitres ou plus.

12. Méthode selon l'une quelconque des revendications 5 à 11, dans laquelle le volume de l'échantillon est de 50 millilitres ou plus.

13. Méthode selon l'une quelconque des revendications 1 à 9, dans laquelle l'échantillon comprend des cellules de sang ou du plasma ou un ou plusieurs embryons ou du sperme.

14. Dispositif destiné à modifier la température d'un échantillon depuis une température initiale en passant par une température intermédiaire jusqu'à une température finale, l'une parmi les températures initiale et finale étant au-dessus du point de congélation dudit échantillon et l'autre étant en dessous du point de congélation, la dimension minimale de l'échantillon dans chacune de deux sections transversales mutuellement perpendiculaires dépassant 0,5 centimètre, ledit dispositif comprenant :
- une piste ;
- un mécanisme destiné à déplacer l'échantillon dans une direction le long de ladite piste ;
- un moyen de contrôle de la température pour imposer un gradient de température le long d'une première zone le long de ladite piste ;
- un moyen de contrôle de la température pour imposer une température constante le long d'une deuxième zone le long de ladite piste, de sorte que la longueur de ladite deuxième zone le long de la piste suffise à permettre à l'échantillon, au niveau de chaque section transversale prise perpendiculairement à ladite direction, d'atteindre la température intermédiaire au moment où il est déplacé hors de ladite deuxième zone ; et
- un moyen de contrôle de la température pour imposer un gradient de température le long d'une troisième zone de ladite piste.

15. Dispositif selon la revendication 14, dans lequel l'échantillon présente un volume d'au moins 5 millilitres.

16. Dispositif selon la revendication 14 ou 15, dans lequel l'échantillon présente un volume dépassant 12 millilitres.

17. Dispositif selon l'une quelconque des revendications 14 à 16, dans lequel l'échantillon présente un volume dépassant 50 millilitres.

18. Dispositif selon la revendication 14, dans lequel le mécanisme de déplacement de l'échantillon le long de ladite piste est capable de déplacer l'échantillon à une vitesse constante le long de la piste dans lesdites première et troisième zones.

19. Dispositif selon la revendication 14, dans lequel le mécanisme de déplacement de l'échantillon le long de ladite piste est capable de déplacer l'échantillon le long de la piste dans lesdites première et troisième zones à des vitesses différentes les unes des autres.

20. Dispositif selon la revendication 14, dans lequel le mécanisme de déplacement de l'échantillon le long de ladite piste est capable d'interrompre le mouvement lorsque l'échantillon est à l'intérieur de la deuxième zone et de reprendre par la suite le mouvement.

21. Dispositif selon l'une quelconque des revendications 14 à 20, dans lequel la température initiale est au-dessus du point de congélation de l'échantillon et la température finale est en dessous dudit point de congélation.
